# FASCICULE DE BREVET EUROPEEN

(11) **EP 3 065 749 B1**
(45) Date de publication et mention de la délivrance du brevet: **24.06.2020**
(21) Numéro de dépôt: 14808522.8
(22) Date de dépôt: 07.11.2014
(51) Int. Cl.: A61K 36/064, A61P 3/04, A61P 3/08, A61P 1/16

(54) **COMPOSITION PHARMACEUTIQUE COMPRENANT SACCHAROMYCES BOULARDII POUR LA REDUCTION DE LA MASSE ADIPEUSE**
PHARMAZEUTISCHE ZUSAMMENSETZUNG ENTHALTEND SACCHAROMYCES BOULARDII ZUR KÖRPERFETTREDUZIERUNG
PHARMACEUTICAL COMPOSITION COMPRISING SACCHAROMYCES BOULARDII FOR REDUCING BODY FAT

(30) Priorité: 08.11.2013 EP 13306537
(43) Date de publication de la demande: 14.09.2016
(73) Titulaire: Biocodex, 94250 Gentilly (FR)
(72) Inventeur: CANI, Patrice D., B-1200 Bruxelles (BE); EVERARD, Amandine, B-1340 Ottignies (BE); GEURTS, Lucie, B-1030 Bruxelles (BE); FARGIER, Emilie, F-60200 Compiegne (FR); VERLEYE, Marc, F-60190 Remy (FR); LE GUERN, Marie-Emmanuelle, F-Compiegne 60200 (FR)
(74) Mandataire: Vial, Lionel
(86) Numéro de dépôt international: PCT/EP2014/074109
(87) Numéro de publication internationale: WO 2015/067788

(56) Documents cités:
- WO-A2-2013/017650
- US-A1- 2006 140 973
- US-A1- 2010 196 413
- KIM K M ET AL: "Yeast hydrolysate reduces body fat of dietary obese rats", PHYTOTHERAPY RESEARCH, JOHN WILEY & SONS LTD. CHICHESTER, GB, vol. 18, 1 janvier 2004 (2004-01-01), pages 950-953, XP003014251, ISSN: 0951-418X, DOI: 10.1002/PTR.1582
- MCFARLAND L V: "Saccharomyces boulardii is not Saccharomyces cerevisiae [4]", CLINICAL INFECTIOUS DISEASES 1996 US, vol. 22, no. 1, 1996, pages 200-201, XP002722451, ISSN: 1058-4838

## Description

### Domaine de l'invention

La présente invention concerne une composition pharmaceutique utile pour réduire la masse adipeuse, notamment chez les individus présentant un excès de masse adipeuse.

### Arrière-plan technique

L'excès de masse adipeuse est en augmentation constante dans la population générale. Ainsi, la proportion des personnes en surpoids ou obèses, respectivement définies par un index de masse corporelle (IMC) supérieur à 25 kg/m² et à 30 kg/m² a progressé de 36,7% à 41,6% entre 1997 et 2003 en France. En outre, la prévalence de l'obésité y est de 14,5% en 2009. Par ailleurs, l'excès de masse adipeuse est un facteur de risque pour de nombreux troubles pathologiques, dont notamment les accidents cardiovasculaires, rendant d'autant plus urgente sa prise en charge thérapeutique.

Si les principaux axes de lutte contre cette épidémie restent l'amélioration de l'alimentation et l'augmentation de la dépense physique des individus présentant un excès de masse adipeuse, d'autres approches, notamment chirurgicales et médicamenteuses sont également explorées.

Les approches médicamenteuses actuelles visent principalement à réduire les apports énergétiques, par exemple en limitant l'absorption intestinale de lipides (orlistat), ou en diminuant l'appétit, soit en favorisant la sensation de satiété (sibutramine), soit en diminuant le plaisir associé à la prise alimentaire (rimonabant).

Toutefois, l'efficacité limitée de ces méthodes ou leurs effets secondaires - il a ainsi été rapporté que le rimonabant pouvait provoquer des troubles dépressifs sévères - ont notamment conduit au retrait de la sibutramine et du rimonabant du marché, laissant des options thérapeutiques très limitées et rendant nécessaires l'exploration d'autres voies.

*Saccharomyces boulardii* (Ultra-Levure®) est une souche particulière de la levure *Saccharomyces cerevisiae.* Ce probiotique est principalement indiqué en complément de la réhydratation pour le traitement des diarrhées. Son utilité a été notamment établie chez l'enfant (Villarruel et al. (2007) Acta Paediatr 96:538-541 ; Szajewska et al. (2007) Aliment Pharmacol Ther 25:257-264) et pour les diarrhées liées à la prise d'antibiotiques (Surawicz et al. (1989) Gastroenterology 96:981-988; Kotowska et al. (2005) Aliment Pharmacol Ther 21:583-590) ou aux infections à *Clostridium difficile* (Surawicz et al. (2000) Clin Infect Dis 31:1012-1017). Par ailleurs, il a été montré que *Saccharomyces boulardii* permettait de prévenir ou de traiter la perte de poids chez des individus souffrant de maladies inflammatoires chroniques de l'intestin et que cet effet est différent de ses effets anti-diarrhéiques (EP 1 693 064 B1).

### Résumé de l'invention

La présente invention découle de la mise en évidence inattendue que l'administration de *Saccharomyces boulardii* à des souris obèses permettait de diminuer la masse adipeuse de ces souris.

Ainsi, la présente invention concerne une composition pharmaceutique comprenant, à titre de substance active, des cellules de levure *Saccharomyces boulardii,* pour son utilisation pour réduire la masse adipeuse chez un individu obèse.

La présente invention concerne également des cellules de levure *Saccharomyces boulardii* pour leur utilisation pour réduire la masse adipeuse chez un individu obèse.

La présente invention concerne également l'utilisation non thérapeutique de cellules de levure *Saccharomyces boulardii* pour réduire la masse adipeuse d'un sujet ne présentant pas d'excès de masse adipeuse ou pour réduire la masse corporelle d'un sujet qui ne présente pas de surpoids ou d'obésité.

Dans un mode de réalisation particulier de la composition pharmaceutique définie ci-dessus, des cellules de levure pour leur utilisation définie ci-dessus et de l'utilisation définie ci-dessus, les cellules de levure *Saccharomyces boulardii* ne sont pas associées ou combinées à de la super oxyde dismutase (SOD) et/ou à un probiotique, en particulier du genre *Lactobacillus* et plus particulièrement de l'espèce *Lactobacillus rhamnosus.* Par ailleurs, dans un mode de réalisation particulier de la composition pharmaceutique définie ci-dessus, des cellules de levure pour leur utilisation définie ci-dessus et de l'utilisation définie ci-dessus, les cellules de levure *Saccharomyces boulardii* ne sont pas enrichies en sélénium.

### Description détaillée de l'invention

### Individu/Sujet

L'individu ou le sujet selon l'invention est un animal, de préférence un mammifère, et plus préférablement un humain, notamment un homme, une femme ou un enfant.

Comme on l'entend ici, la masse adipeuse ou masse grasse ou encore masse graisseuse représente la masse de la totalité des tissus adipeux blancs de l'organisme de l'individu ou du sujet. La masse corporelle de l'individu ou du sujet selon l'invention peut être déterminée à l'aide d'un pèse personne.

Par ailleurs, on préfère que l'individu présente un excès de masse adipeuse, notamment par rapport à sa stature. Cet excès de masse adipeuse est caractérisé par rapport à la normale et peut être évalué par de nombreuses méthodes bien connues de l'homme du métier, notamment par résonance magnétique nucléaire (RMN), en calculant l'index de masse corporelle (IMC) de l'individu (poids corporel exprimé en kg rapporté au carré de la taille exprimé en mètres) ou encore en mesurant le périmètre abdominal de l'individu.

Ainsi, on préfère en particulier que l'individu selon l'invention soit en surpoids ou soit obèse. Selon une définition habituelle un individu humain est considéré en surpoids si son IMC est supérieur ou égal à 25 kg/m² et inférieur à 30 kg/m² et l'individu sera dit obèse si son IMC est supérieur ou égal à 30 kg/m². L'individu selon l'invention peut notamment présenter une obésité sévère, notamment caractérisée chez l'humain par un IMC supérieur ou égal à 35 kg/m².

Plus généralement, on préfère que l'individu selon l'invention soit un humain et présente un IMC supérieur ou égal à 25 kg/m², 26 kg/m², 27 kg/m², 28 kg/m², 29 kg/m², 30 kg/m², 31 kg/m², 32 kg/m², 33 kg/m² kg/m², 34 kg/m², 35 kg/m² ou 40 kg/m² kg/m².

Par ailleurs, l'individu selon l'invention peut également présenter une obésité abdominale, qui correspond en particulier à un excès de tissu adipeux viscéral. Selon une définition habituelle un individu humain masculin présente une obésité abdominale si le périmètre abdominal est supérieur ou égal à 94 cm, en particulier supérieur à 102 cm et un individu humain féminin présente une obésité abdominale si le périmètre abdominal est supérieur ou égal à 80 cm, en particulier supérieur à 88 cm. La mesure du périmètre abdominal est bien connue de l'homme du métier : on mesure ainsi de préférence chez un individu debout et en expiration douce le tour de l'abdomen à mi-distance entre la dernière côte flottante et la partie supérieure de la crête iliaque.

On préfère notamment que l'individu selon l'invention soit un homme et présente un périmètre abdominal supérieur ou égal à 90 cm, 91 cm, 92 cm, 93 cm, 94 cm, 95 cm, 96 cm, 97 cm, 98 cm, 99 cm, 100 cm, 101 cm ou 102 cm. On préfère aussi que l'individu selon l'invention soit une femme et présente un périmètre abdominal supérieur ou égal à 75 cm, 76 cm, 77 cm, 78 cm, 79 cm, 80 cm, 81 cm, 82 cm, 83 cm, 84 cm, 85 cm, 86 cm, 87 cm ou 88 cm.

On préfère également que lorsque l'individu selon l'invention présente un excès de masse adipeuse, qui peut se manifester par un surpoids ou une obésité, notamment abdominale, cet excès de masse adipeuse soit installé, c'est-à-dire qu'il est présent au début du traitement par les cellules de levure *Saccharomyces boulardii* selon l'invention.

On préfère en outre que l'individu selon l'invention soit atteint d'un diabète de type 2 et/ou d'un syndrome métabolique.

On préfère par ailleurs que l'individu selon l'invention présente de plus une stéatose hépatique que les cellules de levure *Saccharomyces boulardii* permettent de traiter. De même, on préfère que l'individu selon l'invention présente de plus une inflammation hépatique et/ou systémique que les cellules de levure *Saccharomyces boulardii* permettent de traiter.

De surcroit, on préfère que l'administration selon l'invention des cellules de levure *Saccharomyces boulardii* à l'individu selon l'invention ne modifie pas significativement la quantité totale de bactéries présentes dans l'intestin de l'individu. Cette quantité peut notamment être évaluée en quantifiant le nombre d'unités formant colonie (UFC) par g de selles de l'individu.

Par ailleurs, on préfère, selon l'invention, que les cellules de *Saccharomyces boulardii* permettent de réduire ou de diminuer la masse corporelle de l'individu selon l'invention, notamment lorsque l'individu selon l'invention est en surpoids ou est obèse.

### Utilisation non thérapeutique

Dans le cadre de l'utilisation non thérapeutique de cellules de levure *Saccharomyces boulardii* pour réduire la masse adipeuse d'un sujet selon l'invention, celui-ci ne présente pas d'excès de masse adipeuse, c'est-à-dire que le sujet ne présente pas de pathologies liées à un excès de masse adipeuse ou n'est pas considéré à risque de développer de telles pathologies. De la même manière, dans le cadre de l'utilisation non thérapeutique de cellules de levure *Saccharomyces boulardii* pour réduire la masse corporelle d'un sujet selon l'invention, celui-ci ne présente pas de surpoids ou d'obésité, c'est-à-dire que le sujet ne présente pas de pathologies liées à un surpoids ou une obésité ou n'est pas considéré à risque de développer de telles pathologies. Ainsi, la réduction de la masse adipeuse ou de la masse corporelle du sujet n'est pas associée à une amélioration de son état de santé et n'a pas de vertu prophylactique. L'utilisation non thérapeutique selon l'invention est alors assimilable à une utilisation cosmétique. De préférence, le sujet ne présentant pas d'excès de masse adipeuse selon l'invention ou ne présentant pas de surpoids ou d'obésité selon l'invention présente ainsi un IMC inférieur à 25 kg/m² et/ou un périmètre abdominal inférieur à 90 ou 94 cm s'il s'agit d'un homme et inférieur à 80 cm s'il s'agit d'une femme.

### Cellules de levure

Comme on l'entend ici, l'expression « cellules de levure » regroupe des cellules de levure viables ou mortes, entières ou sous forme de débris. De préférence, au moins une partie des cellules de levure *Saccharomyces boulardii* selon l'invention sont viables, notamment viables et cultivables, et plus préférablement une majorité des cellules de levures *Saccharomyces boulardii* selon l'invention sont viables, notamment viables et cultivables. De préférence, les cellules de levure *Saccharomyces boulardii* selon l'invention ne sont pas sous forme hydrolysée. De préférence également, l'expression « cellules de levure » ne désigne pas une composition de parois cellulaires de levure.

La viabilité d'une cellule de levure peut notamment être déterminée par coloration au bleu de méthylène et observation microscopique. Le nombre de cellules viables et cultivables, ce qui définit la vitalité, peut être estimé en déterminant le nombre d'Unités Formant Colonie (UFC) comprises dans l'échantillon.

A titre d'exemple, le nombre d'UFC de cellules de levure d'un échantillon liquide comprenant des levures peut être déterminé en étalant un volume déterminé de l'échantillon sur un milieu solide, par exemple gélosé, permettant la croissance des levures et en incubant le milieu solide pendant une durée, par exemple 48 h, et à une température, par exemple 30°C, permettant la croissance de colonies de levures. Le nombre de colonies rapporté au volume étalé sur le milieu solide permet de déterminer le nombre d'UFC compris dans l'échantillon. Un protocole détaillé de la détermination d'UFC conforme à l'invention est notamment décrit dans Toothaker & Elmer (1984) Antimicrobial Agents and Chemotherapy 26:552-556 au paragraphe « *Assay for S. boulardii* ». Par ailleurs, lorsque l'échantillon de levure se présente sous forme d'un solide, par exemple une poudre lyophilisée, on préfère déterminer le nombre d'UFC compris dans l'échantillon après avoir repris une masse déterminée de l'échantillon dans une solution aqueuse, notamment de l'eau distillée ou une solution à 0,9% de NaCl à pH 7.

Ainsi, de préférence, les cellules de levure *Saccharomyces boulardii* selon l'invention comprennent de l'ordre de 10⁸ UFC/g à 10¹² UFC/g, plus préférablement de l'ordre de 2.10⁹ UFC/g à 2.10¹¹ UFC/g.

Une « levure » selon l'invention est un champignon de préférence unicellulaire. Les cellules de levure selon l'invention sont de l'espèce *Saccharomyces boulardii. Saccharomyces boulardii* est bien connu de l'homme du métier et est notamment décrit dans Hennequin et al. (2001) J. Clin. Microbiol. 39:551-559.

De manière particulièrement préférée, les cellules de levure *Saccharomyces boulardii* selon l'invention sont obtenues à partir de médicaments de marque Ultra-Levure®, Bioflor®, Codex®, Econorm®, Enflor®, Enterol®, Florastor®, Floratil®, Florestor®, Inteflora®, Perenterol®, Perenteryl®, Precosa®, Reflor®, Ultra-Levura®, ou à partir des dépôts effectués à *l'American Type Culture Collection* (ATCC, USA) sous la référence 74012 ou à la Collection Nationale de Culture et de Microorganismes (CNCM, France) sous la référence I-745.

De préférence, également les cellules de levure *Saccharomyces boulardii* selon l'invention sont lyophilisées, telles que les cellules de levure *Saccharomyces boulardii* de marque Ultra-Levure®, Bioflor®, Codex®, Econorm®, Enflor®, Enterol®, Florastor®, Floratil®, Florestor®, Inteflora®, Perenterol®, Perenteryl®, Precosa®, Reflor®, ou Ultra-Levura®.

De manière avantageuse, la viabilité et la vitalité de cellules de levure obtenues à partir de lyophilisats sont supérieures à ce qui peut être obtenu à partir d'autres modes de conservation des cellules de levure.

Comme on l'entend ici, la « lyophilisation » est une méthode de conservation dans laquelle les cellules de levure sont congelées puis soumises à une sublimation de l'eau congelée qu'elles contiennent pour donner un lyophilisat sous forme d'une poudre de levure sèche contenant de préférence moins de 2% d'eau et plus préférablement moins de 1% d'eau. De préférence, les cellules de levure lyophilisées sont obtenues à partir de concentrés de cellules de levure. N'importe quel type de méthode de lyophilisation de cellules de levure connu de l'homme du métier peut être utilisé. Toutefois, les cellules de levure sont de préférence lyophilisées selon l'invention à l'aide de la méthode de lyophilisation suivante :
- cultiver les cellules de levure dans un milieu nutritif liquide jusqu'à ce que les cellules atteignent une phase stationnaire;
- concentrer les cellules de levure cultivées et congeler le concentré;
- lyophiliser le concentré.

De préférence, les cellules de levure *Saccharomyces boulardii* sont administrées par la voie orale. Ainsi, les cellules de levure *Saccharomyces boulardii* selon l'invention sont de préférence sous forme de gélules ou de sachets.

De préférence également, les cellules de levure *Saccharomyces boulardii* selon l'invention sont administrées à une dose de 2,5.10⁶ à 5.10¹² UFC/kg/j ou à une dose de 0,00125 g/kg/j à 25 g/kg/j.

Par ailleurs, la composition pharmaceutique telle que définie ci-dessus comprend de préférence une dose de 50 mg à 1000 mg, notamment de 50 mg à 250 mg, de cellules de levure *Saccharomyces boulardii.* En outre, les cellules de levure *Saccharomyces boulardii* pour leur utilisation telle que définie ci-dessus, ou lorsqu'elles sont utilisées de façon non thérapeutique comme cela est défini ci-dessus, sont de préférence administrées à une dose unitaire de 50 mg à 1000 mg, notamment de 50 mg à 250 mg.

Comme l'homme du métier le comprendra, lorsque la quantité de cellules de levure à administrer est exprimée en unité de masse (mg ou g), les cellules de levure sont de préférence sous forme lyophilisée.

Par ailleurs, les cellules de levure *Saccharomyces boulardii* peuvent être associées ou combinées à un ou plusieurs véhicules ou excipients pharmaceutiquement acceptables.

### Description des figures

Figure 1A, Figure 1B, Figure 1C, Figure 1D et Figure 1E

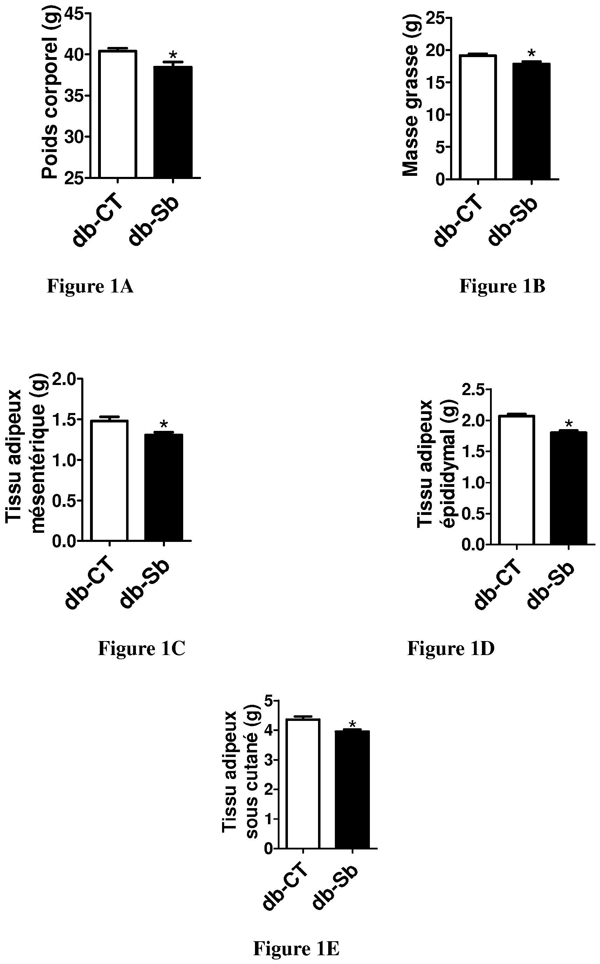

   Les figures 1A-1E représentent respectivement le poids corporel, la masse grasse totale (mesurée par résonance magnétique nucléaire), la masse grasse mésentérique, la masse grasse épididymale et la masse grasse sous-cutanée exprimés en gramme (g) de souris obèses diabétiques témoins (colonnes blanches, db-CT) ou ayant reçu une administration orale de cellules de levure *Saccharomyces boulardii* (colonnes noires, db-Sb). Le symbole * représente une différence significative (P<0,05) des souris traitées par rapport aux souris témoins.
Figure 2

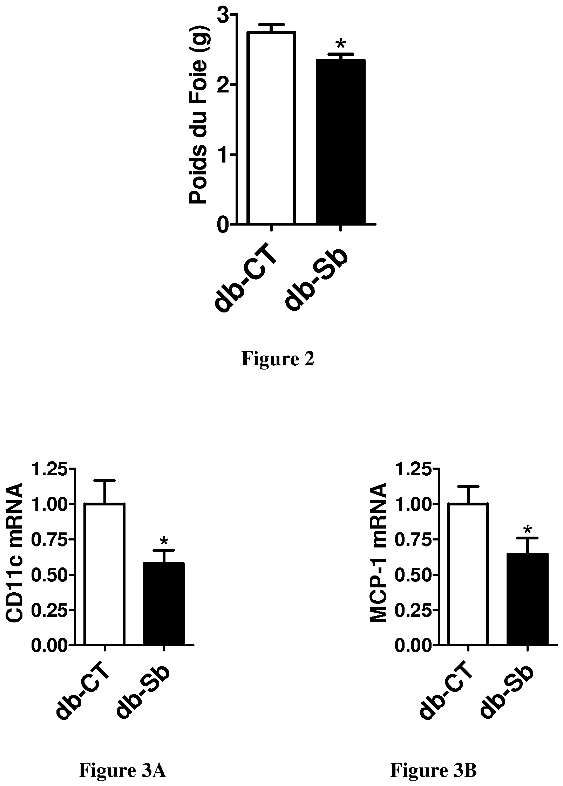

   La figure 2 représente le poids du foie exprimé en gramme (g) de souris obèses diabétiques témoins (colonnes blanches, db-CT) ou ayant reçu une administration orale de cellules de levure *Saccharomyces boulardii* (colonnes noires, db-Sb). Le symbole * représente une différence significative (P<0,05) des souris traitées par rapport aux souris témoins.
Figure 3A, Figure 3B, Figure 3C et Figure 3D
   Les figures 3A-3D représentent respectivement la quantité d'ARNm de CD11c, de MCP-1, d'IL-1β et de F4/80 dans le foie de souris obèses diabétiques témoins (colonnes blanches, db-CT) ou ayant reçu une administration orale de cellules de levure *Saccharomyces boulardii* (colonnes noires, db-Sb). Le symbole * représente une différence significative (P<0,05) des souris traitées par rapport aux souris témoins.
Figure 4

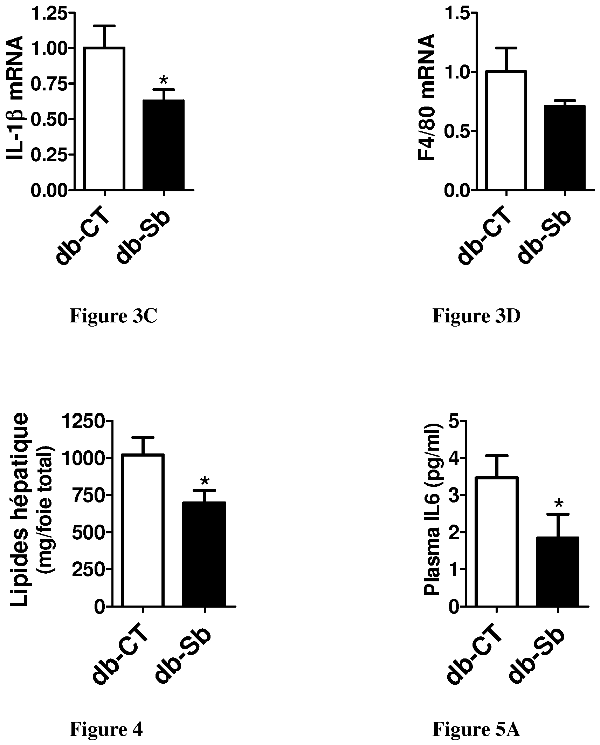

   La figure 4 représente la masse lipidique contenue dans le foie (exprimée en mg de foie entier) de souris obèses diabétiques témoins (colonnes blanches, db-CT) ou ayant reçu une administration orale de cellules de levure *Saccharomyces boulardii* (colonnes noires, db-Sb). Le symbole * représente une différence significative (P<0,05) des souris traitées par rapport aux souris témoins.
Figures 5A, 5B et 5C

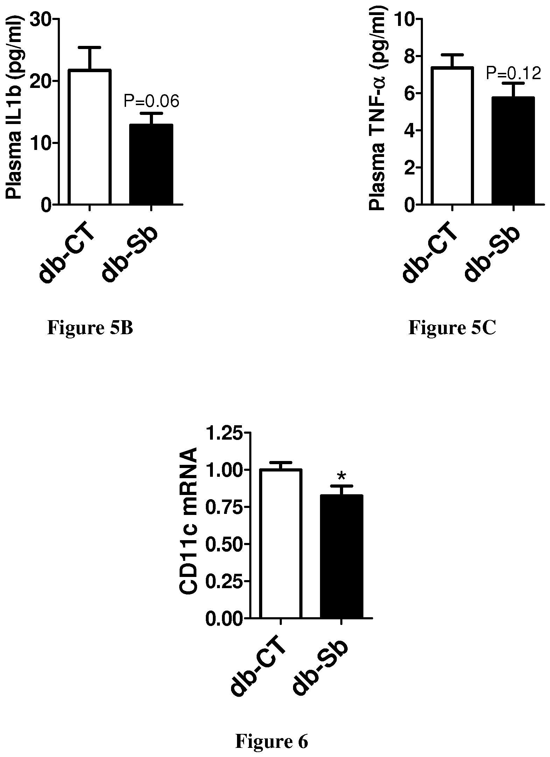

   Les figures 5A-5C représentent respectivement la quantité plasmatique d'IL-6, d'IL-1β et de TNF-α de souris obèses diabétiques témoins (colonnes blanches, db-CT) ou ayant reçu une administration orale de cellules de levure *Saccharomyces boulardii* (colonnes noires, db-Sb). Le symbole * représente une différence significative (P<0,05) des souris traitées par rapport aux souris témoins.
Figure 6
   La figure 6 représente la quantité d'ARNm de CD11c dans le colon de souris obèses diabétiques témoins (colonnes blanches, db-CT) ou ayant reçu une administration orale de cellules de levure *Saccharomyces boulardii* (colonnes noires, db-Sb). Le symbole * représente une différence significative (P<0,05) des souris traitées par rapport aux souris témoins.
Figures 7A, 7B, 7C, 7D, 7E et 7F

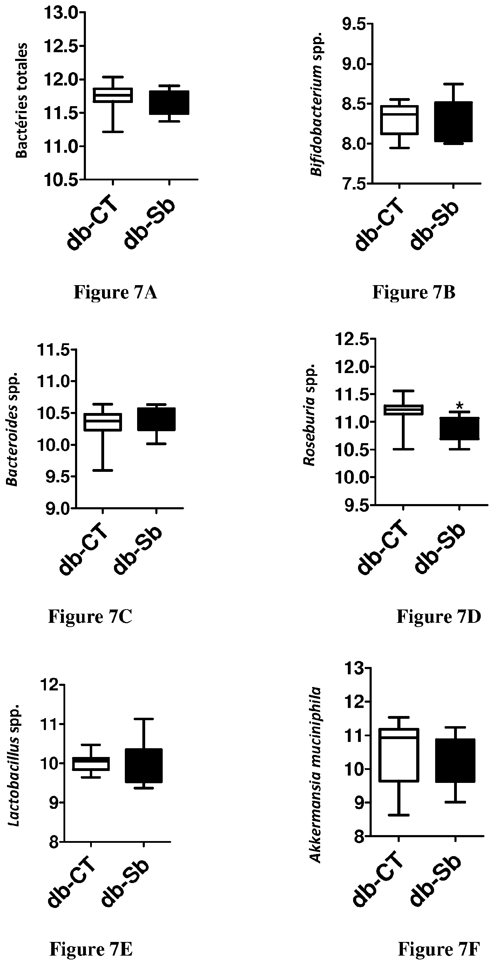

   Les figures 7A-7F représentent respectivement la quantité de bactéries totales et de bactéries *Bifidobacterium* spp., *Bacteroides* spp., *Roseburia* spp, *Lactobacillus* spp. et *Akkermansia muciniphila,* dans le caecum de souris obèses diabétiques témoins (colonnes blanches, db-CT) ou ayant reçu une administration orale de cellules de levure *Saccharomyces boulardii* (colonnes noires, db-Sb) exprimée en log₁₀ de la quantité de bactéries par gramme de contenu caecal. Le symbole * représente une différence significative (P<0,05) des souris traitées par rapport aux souris témoins.

### Exemple

### 1. Protocole expérimental

30 souris génétiquement obèses et diabétiques de type 2 db/db *(BKS.Cg-Dock7m* +/+*Lep db*/J) (Charles river) âgées de 6 semaines ont reçu quotidiennement, par gavage oral, la levure *Saccharomyces boulardii* (Ultralevure, Biocodex) (3g/kg) (db-Sb) ou le véhicule (NaCl 0.9%) (db-CT) (n=15 souris/groupe).

Différents paramètres ont été évalués lors de l'expérience :
**a)** Le poids corporel.
**b)** Le poids de différents tissus et organes après prélèvement : foie, colon et différents tissus adipeux (viscéral (mésentérique), épididymal et sous-cutané).
   Les prélèvements de tissus ont été effectués en fin d'expérience, les souris ont alors été mises à jeun 5 h avant sacrifice. Les souris ont d'abord été anesthésiées avec de l'isoflurane (Isoba, Schering-Plough Animal Health, Uxbridge, Middlesex, Royaume-Uni), puis du sang a été prélevé dans la veine cave ainsi que dans la veine porte, et enfin elles ont été euthanasiées par dislocation cervicale. Différents tissus et organes ont été prélevés: foie, colon et différents tissus adipeux (viscéral (mésentérique), épididymal et sous-cutané).
**c)** La masse grasse.
   La masse grasse a été mesurée en fin d'expérience par RMN *(time-domain* résonance magnétique nucléaire (TD-RMN) (Bruker Minispec mq 7.5 NMR Analyzer, The Bruker Corporation, Billerica, MA, USA).
**d)** Plusieurs organes ont été analysés (par qPCR) afin d'étudier différents marqueurs de l'inflammation et de l'infiltration des macrophages (CDllc, IL-1β, F4/80, MCP-1).
   Pour le colon, le tissu adipeux sous-cutané et le tissu adipeux mésentérique, une partie du tissu ou de l'organe en question a été plongée dans une solution de *RNA later* (Applied Biosystems, Californie, USA) pendant 1 semaine à 4°C afin de préserver la qualité de l'ARN. Après ce traitement, les tissus et organes ont été stockés à -80°C. Le reste des tissus et organes a été congelé dans de l'azote liquide pour être finalement stocké dans un congélateur à -80°C.
   L'ARN des tissus a été extrait à l'aide d'une solution de tripure (Tripure Isolation Reagent, Roche, Mannheim, Suisse) après homogénéisation à l'aide d'un *Tissue Lyser* (Qiagen, Hilden, Allemagne) à 30 Hz pendant 4 minutes. Une centrifugation de 10 min à 4 °C et à 12 000 g a permis d'éliminer les débris tissulaires. L'intégrité de l'ARN extrait ainsi que sa concentration ont été mesurées au *bioanalyser* grâce au kit *RNA 6000 Nano* (Agilent Technologies, Santa Clara, USA). L'intégrité des ARN extraits des tissues et organes a été mesurée grâce au RIN (*RNA Integrity Number*). Ensuite, une transcription inverse a été réalisée à partir de 1µg d'ARN extrait. (*Reverse Transcription System,* Promega Corporation, Madison, Misconsin, USA). Les PCR quantitatives ont été réalisées à l'aide du kit *MESA FAST qPCR SYBR Green* (Eurogentec, Seraing, Belgique) en microplaques. Les microplaques ont été analysées en temps réel à l'aide d'un appareil qPCR de marque StepOnePlus (Applied Biosystems, Foster city, Californie, USA). Les quantités d'amplicons des différents gènes marqueurs de l'inflammation et de l'infiltration des macrophages (CD11c, IL1β, F4/80, MCP-1) ont ensuite été rapportées aux quantités d'amplicons d'un gène témoin codant pour la protéine ribosomiale L19 ou RPL19.
**e)** Le contenu en lipides totaux du foie.
   Le contenu en lipides totaux du foie a été quantifié par gravimétrie après extraction des lipides par la méthode de Folch (Folch et al. (1957) J. Biol. Chem. 226:497) à l'aide d'une solution contenant du chloroforme et du méthanol dans un rapport de 2 pour 1 (Solution de Folch).
**f)** Le microbiote intestinal a été analysé par qPCR après extraction de l'ADN (QIAamp-DNA stool mini-kit (Qiagen, Hilden, Germany) pour les taxa suivants :
   a. Bactéries totales.
   b. *Bifidobacterium* spp.
   c. *Lactobacillus* spp.
   d. *Bacteroides*/*Prevotella* spp.
   *e. Akkermansia muciniphila.*
   f. *Roseburia* spp.
   Chaque taxa a été quantifié sur base d'une droite d'étalonnage contentant une quantité précise d'ADN et correspondant à un nombre de cellules cultivées et quantifiées.
g) La mesure de la concentration plasmatique des cytokines IL-1β, TNFα, et IL-6 a été réalisée à l'aide de la technologie du Luminex (BioPlex 200, BioRad) à l'aide d'un kit synthétisé à la demande par la société Millipore (Milliplex, Millipore, Belgique).

L'analyse statistique des données a été réalisée à l'aide d'un test de Student, avec un seuil de significativité de P<0,05.

### 2. Résultats

### 2.1. Poids corporel, masse grasse mesurée par RMN et poids des différents tissus adipeux

Le traitement par S. *boulardii* diminue de façon significative le poids corporel (Figure 1A), l'accumulation de graisse mesurée par résonance magnétique nucléaire (Figure 1B) et les tissus adipeux blancs (Figures 1C, 1D, 1E).

### 2.2. Poids des organes aux sacrifices

Le traitement par S. *boulardii* diminue significativement le poids du foie (-15%) (Figure 2).

### 2.3. Marqueurs de l'inflammation du foie

Le traitement par S. *boulardii* diminue de manière significative l'infiltration des macrophages, illustrée par une diminution du niveau d'expression des ARNm des marqueurs d'infiltration et recrutement des macrophages CD11c, MCP-1, F4/80 (Figures 3A, 3B et 3D), et l'inflammation hépatique, illustrée par une diminution du niveau d'expression de l'ARNm de la chaine β de la cytokine inflammatoire IL-1 (Figure 3C).

### 2.4. Contenu lipidique du foie

Le traitement par S. *boulardii* diminue de 30% le contenu en lipides totaux du foie (Figure 4).

### 2.5. Cytokines circulantes

Différentes cytokines plasmatiques ont été mesurées dans le plasma de la veine cave à l'aide de la technologie du Luminex. Le traitement avec *S*. *boulardii* induit une modification importante des paramètres immunitaires circulants. En effet, les taux plasmatiques de l'interleukine inflammatoire IL-6 sont significativement diminués, par ailleurs deux autres cytokines considérées comme « pro-inflammatoires » (IL-1β et TNFα) tendent à être diminuées.

### 2.6. Colon

Le traitement diminue de façon significative l'infiltration des macrophages dans le colon (Figure 6).

### 2.7. Analyse du microbiote intestinal

Il a été procédé à une quantification par qPCR des bactéries totales (Figure 7A) et de différents genres bactériens connus comme ayant un potentiel impact sur le métabolisme glucidique, lipidique et la fonction de barrière de l'intestin, à savoir : *Bifidobacterium* spp. (Figure 7B), *Bacteroides* spp. (Figure 7C), *Roseburia* spp. (Figure 7D), *Lactobacillus* spp. (Figure 7E) et *Akkermansia muciniphila* (Figure 7F).

L'administration de *S*. *boulardii* ne modifie pas de manière significative la quantité totale de bactéries dans l'intestin. De fait, il n'y a pas de modification significative des bactéries des différents taxa investigués a l'exception de *Roseburia* spp. (Figure 7D).

Ceci suggère que *S*. *boulardii* exercerait un impact direct sur le métabolisme de l'hôte et potentiellement ne nécessitant pas l'intervention d'autres bactéries.

### Conclusion générale

Les souris obèses et diabétiques traitées avec *S*. *boulardii* présentent une diminution significative de la masse grasse (diminuée d'environ 10%). Cette diminution de la masse grasse contribue à la baisse du poids corporel (-5%). Par ailleurs, l'administration de *S. boulardii* n'entraîne pas de modification notable de la composition du microbiote intestinal (nombre total de bactéries dans l'intestin).En outre, l'administration de S. *boulardii* est associée à une diminution significative du poids du foie probablement expliquée par la diminution très nette du contenu en lipide dans cet organe (diminution de la stéatose hépatique de 30%). De façon intéressante, l'ensemble de ces effets sont associés à une diminution des marqueurs inflammatoires (CD11C, MCP-1, IL-1β) dans le tissu hépatique. Cette diminution de l'inflammation hépatique est également associée à une diminution de différents paramètres inflammatoires systémiques..

En conclusion, l'administration de *S*. *boulardii* diminue significativement la masse grasse et la stéatose hépatique chez la souris obèse et diabétique de type 2 (db/db) ainsi que l'inflammation hépatique et systémique.

## Revendications

1. Composition pharmaceutique comprenant, à titre de substance active, des cellules de levure *Saccharomyces boulardii,* pour son utilisation pour réduire la masse adipeuse chez un individu, dans laquelle l'individu est obèse.

2. Composition pharmaceutique pour son utilisation selon la revendication 1, dans laquelle l'individu présente une obésité abdominale.

3. Composition pharmaceutique pour son utilisation selon la revendication 1 ou 2, dans laquelle l'individu est atteint d'un diabète de type 2.

4. Composition pharmaceutique pour son utilisation selon l'une des revendications 1 à 3, dans laquelle l'individu est atteint d'un syndrome métabolique.

5. Composition pharmaceutique pour son utilisation selon l'une des revendications 1 à 4, pour traiter en outre une stéatose hépatique de l'individu.

6. Composition pharmaceutique pour son utilisation selon l'une des revendications 1 à 5, pour réduire en outre une inflammation hépatique et/ou systémique de l'individu.

7. Composition pharmaceutique pour son utilisation selon l'une des revendications 1 à 6, dans laquelle les cellules de levure sont lyophilisées.

8. Cellules de levure *Saccharomyces boulardii* pour leur utilisation pour réduire la masse adipeuse chez un individu, dans laquelle l'individu est tel que défini dans les revendications 1 à 4.

9. Cellules de levure pour leur utilisation selon la revendication 8, pour traiter en outre une stéatose hépatique de l'individu et/ou pour réduire en outre une inflammation hépatique et/ou systémique de l'individu.

10. Utilisation non thérapeutique de cellules de levure *Saccharomyces boulardii* pour réduire la masse adipeuse d'un sujet ne présentant pas d'excès de masse adipeuse.

## Patentansprüche

1. Pharmazeutische Zubereitung, als Wirkstoff Zellen der Hefe *Saccharomyces boulardii* enthaltend, zum Gebrauch zur Verringerung der Fettmasse eines Patienten, wobei der Patient adipös ist.

2. Pharmazeutische Zubereitung zum Gebrauch nach Patentanspruch 1, wobei der Patient abdominale Adipositas aufweist.

3. Pharmazeutische Zubereitung zum Gebrauch nach Patentanspruch 1 oder 2, wobei der Patient an einer Diabetes Typ 2 leidet.

4. Pharmazeutische Zubereitung zum Gebrauch nach einem der Patentansprüche 1 bis 3, wobei der Patient an einer Stoffwechselkrankheit leidet.

5. Pharmazeutische Zubereitung zum Gebrauch nach einem der Patentansprüche 1 bis 4, um außerdem eine Leberepithelverfettung des Patienten zu behandeln.

6. Pharmazeutische Zubereitung zum Gebrauch nach einem der Patentansprüche 1 bis 5, um außerdem eine Hepatitis und/oder systemische Entzündungsreaktion des Patienten zu behandeln.

7. Pharmazeutische Zubereitung zum Gebrauch nach einem der Patentansprüche 1 bis 6, in der die Hefezellen lyophilisiert sind.

8. Zellen der Hefe *Saccharomyces boulardii,* zum Gebrauch zur Verringerung der Fettmasse eines Patienten, wobei der Patient den Patentansprüchen 1 bis 4 entspricht.

9. Hefezellen zum Gebrauch nach Patentanspruch 8, außerdem zur Behandlung einer Leberepithelverfettung des Patienten und/oder außerdem zur Verringerung einer Hepatitis und/oder systemischen Entzündungsreaktion des Patienten.

10. Nicht-therapeutischer Gebrauch von Zellen der Hefe *Saccharomyces boulardii* zur Verringerung der Fettmasse eines Patienten, der keine übermäßige Fettmasse aufweist.

## Claims

1. Pharmaceutical composition comprising, as active substance, Saccharomyces *boulardii* yeast cells, for use for reducing the adipose mass in an individual, wherein the individual is obese.

2. Pharmaceutical composition for use according to claim 1, wherein the individual has an abdominal obesity.

3. Pharmaceutical composition for use according to claim 1 or 2, wherein the individual has a type 2 diabetes.

4. Pharmaceutical composition for use according to any one of claims 1 to 3, wherein the individual has a metabolic syndrome.

5. Pharmaceutical composition for use according to any one of claims 1 to 4, for further treating a hepatic steatosis of the individual.

6. Pharmaceutical composition for use according to any one of claims 1 to 5, for further reducing hepatic and/or systemic inflammation of the individual.

7. Pharmaceutical composition for use according to any one of claims 1 to 6, wherein the yeast cells are lyophilized.

8. *Saccharomyces boulardii* yeast cells for use for reducing body fat in an individual, wherein the individual is as defined in claims 1 to 4.

9. Yeast cells for use according to claim 8, for further treating a hepatic steatosis of the individual and/or for further reducing hepatic and/or systemic inflammation of the individual.

10. Non-therapeutic use of *Saccharomyces boulardii* yeast cells for reducing the fat mass of a subject who does not have excess of fat mass.
